# EUROPEAN PATENT APPLICATION

(11) **EP 2 186 876 A1**
(43) Date of publication of application: **19.05.2010**
(21) Application number: 08169020.8
(22) Date of filing: 13.11.2008
(51) Int. Cl.: C12M 1/16

(54) **System for solid state fermentation and use thereof**

(71) Applicant: Biorecycling Group B.V., 1525 RJ Westknollendam (NL)
(72) Inventor: Hoogeveen, Gerardus Wilhelmus Maas, 3633 EA Vreeland (NL); Hoogeveen, Hermanus Wilhelmus, 3633 EA Vreeland (NL)
(74) Representative: van Heuvel, Margaretha

(57) **Abstract**

The present invention discloses a system for solid state fermentation of a fungus, the system comprising a plurality of separate fermentation units, wherein the size of at least one dimension of each unit is accommodated to provide conditions for aerobic fermentation of the fungus without the need to install in the unit means for at least one of forced aeration, forced agitation and active cooling of the fungal culture. The system can be used for biodegradation/bioremediation of (polluted) biomass. The size of the at least one dimension is at most about 50 cm.

## Description

The present invention relates to a system for solid state fermentation of a fungus, and use thereof. The present invention further describes a method for solid state fermentation of a fungus using the system.

Disposal and proper processing of biomass waste and polluted biomass is hampered by the diverging composition, quality and contamination level of the various types of biomass.

Bioconversion with fungi using solid state fermentation has been described for degradation of clean biomass, waste biomass as well as contaminated biomass.

Solid state fermentation (SSF) has been defined as a fermentation process occurring in the absence or near-absence of free water. Contrary to submerged fermentation, solid state fermentation currently is of limited industrial application because of the lack of engineering data and knowledge about the design and scale up of solid state fermenters.

WO 99/57239 and WO 00/29544 disclose solid state fermenters which are modular in nature, whereby the modules are placed on top of each other.

The present invention discloses a system for solid state fermentation that is simple, does not require extensive engineering and is easily scaled up to any desired scale.

In a first aspect, the present invention discloses a system for solid state fermentation of a fungus comprising a plurality of units placed next to each other. The system is especially suitable for solid state fermentation of a fungus on a biomass substrate, to obtain degradation and/or remediation of the substrate. The system as described herein, comprising a plurality of (at least two) separate fermentation units, advantageously enables scaling of the system to any desired scale.

The units of the system as described herein are preferably placed in a suitable housing, such as a greenhouse or a cellar. The housing may provide suitable incubation conditions for the fungus, like temperature, relative humidity, light.

A unit of the system for solid state fermentation of a fungus may be a simple container that should have at least one dimension that is accommodated to provide conditions for aerobic fermentation of the fungus without the need to install in the unit means for at least one of forced aeration, forced agitation and active cooling of the fungal culture. In particular, this at least one dimension of the unit should have a size that is accommodated to circumvent heat build-up in the fungal culture present in the unit as a consequence of fungal growth and metabolism as well as to enable air to passively enter the fungal culture inside the unit in order to maintain aerobic metabolism substantially throughout the culture. Preferably, the at least one dimension has a length up to about 50 cm, for instance about 5 to about 50 cm, preferably about 10 to about 40 cm, more preferably about 20 to about 40 cm.

In the context of the invention, a dimension as mentioned herein is a dimension that relates to the internal volumetric dimensions of the unit.

The other dimensions of the unit are less critical and are mainly determined by ease of handling and desired scale. For instance, the unit may have a volume of about 10 to about 150,000 Liter, preferably of about 20 to about 35,000 Liter, more preferably of about 50 to about 10,000 Liter, even more preferably of about 100 to about 3000 Liter. The units may be cylindrical, square, rectangular. Larger units may be provided with one or more partitions creating smaller spaces within a unit. Such partitions may be placed at a distance of e.g about 0.5 to about 2 m from each other, horizontally and/or vertically. The partitions may be made of the same material as the walls of the unit.

The walls of the units may be made of any suitable material, for instance (stainless) steel, aluminium or plastic, provided that at least one wall of the unit is permeable to gas. The gas-permeable wall preferably is a wall with the largest surface area, usually a lateral wall. More preferably, two walls with the largest surface area are gas-permeable. Gas permeability may be provided by using a perforated wall. The perforations may have a diameter of about 0.5 to about 2 cm, preferably of about 0.5 to about 1 cm, and may be placed at a distance of about 0.1 to about 5 cm, preferably of about 1 to about 5 cm, from each other. In one embodiment, the gas-permeable wall is made of wire mesh.

Each unit may be provided with means to load and/or unload the unit. For instance, the unit may be provided with movable upper and/or movable bottom plates. The loading and/or unloading of a unit preferably occurs when a unit occupies at least one specific position in the system. Said at least one specific position configured for loading and/or unloading of the unit may at the most constitute 50% of the total amount of positions in the system, preferably constitute less than 20%, more preferably less than 10%, most preferably less than 5%, of the total amount of positions in the system.

The plurality of units of the system is preferably arranged in parallel rows. Configuration of the rows, i.e. amount of units in a row and amount of rows, can conveniently be varied depending on e.g. the desired scale.

The units are preferably movable in a horizontal plane, more preferably in an endless path. Movement of the units may be achieved by using a rail system placed above and/or beneath the units.

Especially an arrangement of a plurality of movable units containing at least one position configured for loading and/or unloading of a unit advantageously provides the option to move each unit in the plurality of units from a loading position to an unloading position and to establish that the units within the plurality of units are in different stages of the fermentation process. When and/or at which position unloading of a unit occurs may be determined by the stage of the solid state fermentation process in the unit. Such an arrangement provides flexibility and optimal use of the available space.

In one embodiment, a series of movable units is placed on a bottom plate supporting the units, in such a way that the units are movable over the bottom plate. The bottom plate may be one plate or may consist of two or more plates.

The bottom plate preferably is interrupted by holes to enable discharge of the unit. The amount and positioning of the holes in the bottom plate may suitably be chosen depending on the optimal utilization of the volumetric capacity of the incubation space. Discharge of the unit may comprise unloading of the unit as well as removal of the complete unit.

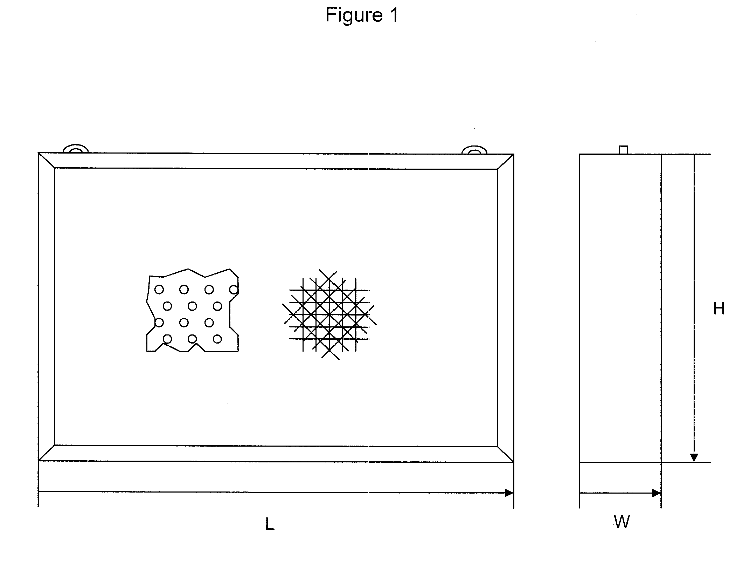

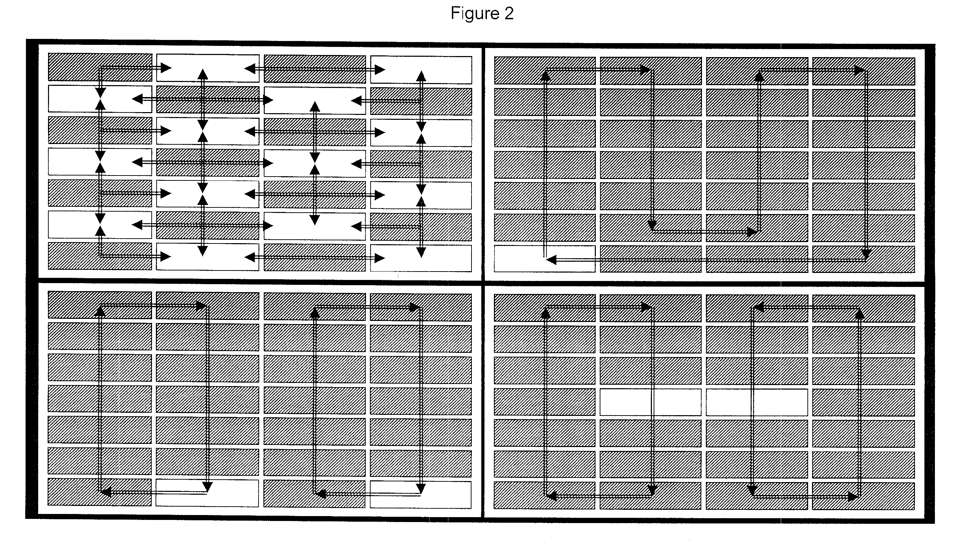

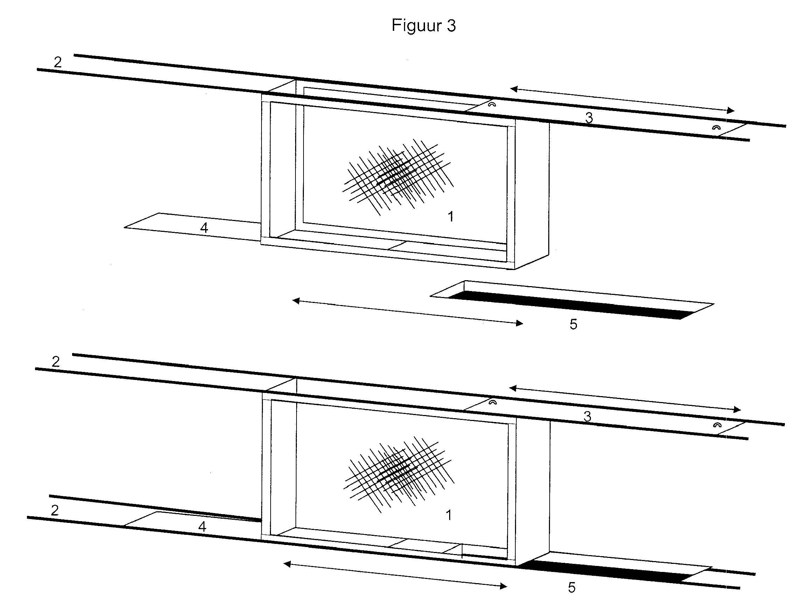

Figures 1 to 3 relate to embodiments of the invention, wherein the fermentation unit is a rectangular unit.
Figure 1 depicts a rectangular unit having a lateral wall made of a perforated plate or wire mesh. The unit has a width W up to about 50 cm, a height H of about 5 cm to about 15 m and a length L of about 5 cm to about 20 m. The height of the unit preferably is about 50 cm to about 7 m, more preferably about 50 cm to about 5 m. The length of a unit preferably is about 50 cm to about 10 m, more preferably about 50 cm to about 5 m.
Figure 2 shows several options for optimal use of the space of a housing wherein the units are placed. The arrows depict the direction of movement of the units. The shaded rectangles represent positions of units and the empty rectangles depict positions for unloading the unit.
Figure 3 schematically shows a unit 1 that is movable using a rail system 2 placed above and/or beneath the unit. The unit contains a movable plate 3 for loading the unit and a movable plate 4 for unloading the unit. The unit is unloaded into a space 5.

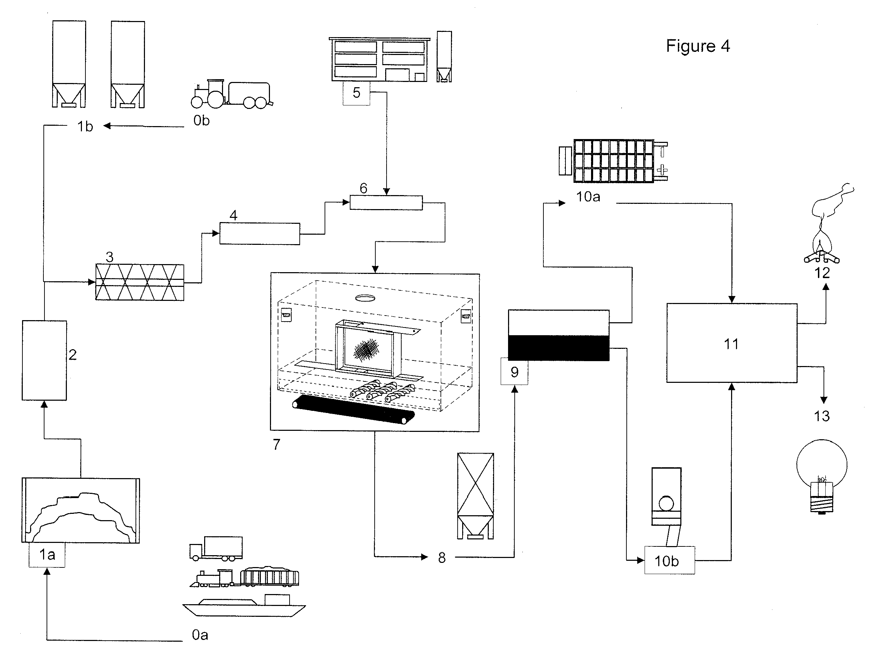

Figure 4 shows an example of a flow scheme of different steps in the processing of biomass using solid state fermentation as described herein. The numbering refers to the steps as indicated below.
   - 0a.: Incoming biomass
   - 0b.: Liquid, e.g. manure
   - 1a.: Storage biomass
   - 1b.: Storage manure
   - 2.: Shredding of biomass to e.g. 0-6 mm
   - 3.: Composing the optimal suspension
   - 4.: Pre-treatment, e.g. pasteurization/sterilization
   - 5.: Fungal library
   - 6.: Inoculation of suspension
   - 7.: Solid state fermentation
   - 8.: Separation into a solid and liquid fraction
   - 9.: Storage of the solid and liquid fraction
   - 10a.: Use of the liquid fraction in a microbial fuel cell
   - 10b.: Pelletizing of the solid fraction
   - 11.: Supply to e.g. energy/power company for e.g.:
   - 12.: Heating
   - 13.: Electricity

In a second aspect, the present invention discloses use of the system of the first aspect for solid state fermentation of a fungus.

In particular, solid state fermentation of the fungus is done by growth of the fungus on a substrate comprising biomass, to achieve biodegradation and/or bioremediation of the biomass-containing substrate. The biomass may be contaminated with organic or inorganic pollutants. Such biodegradation and/or bioremediation of a biomass-containing substrate may advantageously result in a fermentation product that is more suitable for further processing and further use than the starting biomass material.

In the methods as described herein, the biomass may be any suitable biomass material, i.e. biomass of plant, animal and/or microbial origin. Examples of biomass materials are wood, i.e. hard as well as soft wood, like wood cuttings, waste wood, painted wood, saw dust, preserved wood; crop residues like rice or wheat straw, corn stalks, soy residues, wheat bran, nut shells, sugar beet pulp; animal remains like skin, horns, hooves, bone, feathers, meal; paper waste; frying fats; municipal waste and/or agricultural waste.

To ensure that the biomass is a suitable food source for the fungus, it may be necessary to use a mixture of different types of biomass. For instance, if biomass with a low nitrogen content is used, it may be supplemented with biomass material and/or a food source that is high in nitrogen. Examples of high nitrogen food sources are soy meal, corn steep liquor, yeast extract. In addition, a hard wood biomass may be supplemented with soft wood and/or a non-wood biomass. A polluted substrate may further be mixed with a not polluted substrate. If the substrate to be degraded further is relatively poor or even deficient in one or more minerals and/or vitamins, these materials may be supplemented in the process.

The biomass (mixture) to be subjected to the biodegradation/bioremediation as described herein may further be adjusted depending on desired specifications of the end product.

The methods as described herein are especially suitable for bioremediation of contaminated or polluted biomass, such as biomass contaminated with complex organic molecules such as polycyclic aromatic hydrocarbons. An example of contaminated biomass is found among railroad ties impregnated with coal tar creosote.

In one embodiment, the substrate is brought in the form of particles having a diameter of at the most 15 mm, preferably at the most 10 mm, more preferably at the most 5 mm. The lower limit of the particle size may be as low as possible. i.e. dust like. Particles may be obtained by using any suitable shredding technology. In this way, it is possible to have the substrate to be subjected to fungal degradation in the form of a suspension. The substrate being in the form of a suspension advantageously facilitates processing steps, for instance enables simple mixing of the fungal inoculum with the substrate. In addition, the finer the particle size of the suspension, the faster the biodegradation will occur. When a fibrous substrate like wood is used, it is preferred to splice the fibers as much as possible over the length of the fiber.

The substrate optionally may be pre-treated by a heat treatment or UV irradiation prior to incubation with the fungus, to reduce or eliminate undesirable microorganisms. Such a treatment preferably is done on the substrate being in the form of particles. A suitable heat treatment is steam sterilization or flash sterilization. However, an advantage of the methods as described herein is that the substrate does not need to be completely sterile.

The moisture content of the substrate to be subjected to fungal degradation should be adjusted to obtain a dry matter content of 40-90 % (w/w). The liquid used to adjust the moisture content of the substrate may be any suitable liquid, for instance tap water. However, it is preferred to use manure and/or liquid agricultural and/or industrial waste streams.

The substrate may be (partially) dried prior to the addition of manure and/or liquid agricultural and/or industrial waste streams to enable the use of larger volumes of these waste liquids.

In one embodiment, the particulate substrate may be dried to enable storage of the particulate substrate for a time period as desired.

The substrate, preferably in the form of a suspension, is inoculated with an inoculum of a fungus. The fungal species to be used may thereby depend on the nature of the substrate to be degraded.

The different processing steps of the substrate may conveniently be done by passing the substrate through a conveyor provided with means for treatment of the substrate as desired.

The substrate, preferably in particle form, may be fed into a conveyor, then conveyed over a distance on the conveyor and agitated during the conveying step such that the substrate is moved sufficiently to permit access to a substantial portion of the surfaces of the substrate and thereby performing various treatments while the substrate is being conveyed. Finally, the substrate so treated is collected and fed into the unit for solid state fermentation as described herein.

The treatments during the conveying step may include adjusting the moisture content of the substrate by adding moisture or by drying, heating the substrate by passing the substrate through an amount of steam, cooling the substrate to a temperature physiologically suitable for inoculating fungi, and mixing an amount of inoculant with the substrate, the inoculant comprising an inoculating fungus and, optionally, a nutrient adjuvant.

The conveyor thus may be provided with an input source, such as a feeder, for feeding the substrate into the conveyor. In addition, the conveyor may include a means for agitating the substrate such that a substantial portion of the surfaces of the substrate is exposed to treatment. The conveyor further may include a means for heating the substrate and a means for cooling the substrate. Finally, the conveyor may include an inoculator having an amount of fungal inoculum and being capable of dispersing the inoculum through the substrate such that the fungal inoculum may propagate.

For solid state cultivation, a Basidiomycete fungus is especially suitable. The fungus preferably is a wood rot fungus, especially for biomass that comprises wood. Such a fungus also is suitable to degrade complex organic pollutants when present in the substrate to be degraded. Examples of suitable wood rot fungal genera are *Ganoderma, Pleurotus, Lentinus, Bjerkandera, Ceriporiopsis, Phlebia, Ceriporiopsis, Dichomitus, Perenniporia, Hyphodontia, Mycoacia, Phellinus, Rigidoporus, Phanerochaete, Trametes, Meruliporia, Antrodia, Neolentinus,* and *Polyporus.* A single fungal species or a mixture of different fungal species or genera may be used.

The fungal inoculum may be prepared using cultivation techniques generally known in the art. For instance, the fungal inoculum is prepared by first growing the fungus in a culture containing a suitable agar medium, for instance malt extract agar, potato dextrose agar or cornmeal yeast glucose agar. The agar culture is typically incubated for one to two weeks, or until a confluent fungal growth is achieved over the agar surface. After achieving a confluent fungal growth, the fungus is transferred to a liquid culture medium. Suitable liquid media may be a very simple medium, for instance containing 4 % (w/w) glucose and 0.05 % (w/w) calcium carbonate, or a more complex medium, for instance containing 4 % glucose, 0.1 % peptone, 0.2 % yeast extract, 0.1 % K₂HPO₄, 0.02 % MgSO₄.7H₂O and 0.5% (NH₄)₂SO₄. After about 7 days of growth, an up scale of a factor 5 may be done, and this may be repeated depending on the amount of fungus needed. Typically, the fungal inoculum may contain about 10-20 % (w/w) fungal biomass.

The fungal inoculum is mixed with the substrate in an amount of 5-20 % (w/w) of fungal inoculum based on wet weight of substrate. The amount of fungal inoculum thereby may be determined by the nature and composition of the biomass. Hard wood typically may require more fungal inoculum than soft wood or non-wood biomass.

In one embodiment of the invention, the fungus is adapted to degrade a particular substrate by serial transfer and growth of the fungus on the particular substrate, to enrich for strains with an improved growth performance on the substrate.

To adapt a fungus to degrade a particular pollutant, serial transfer preferably is done by growth on an agar culture supplemented with the pollutant. The fungus preferably is serially transferred to cultures with increasing concentrations of the pollutant. Three to five serial transfers may be done, taking 12 to 75 days depending on the growth rate of the fungus. It is also possible to adapt the fungus using a compound or mixture of compounds representative for the pollutant. Such a mixture of compounds for instance may be an extract of a polluted biomass material.

In another embodiment of the invention, the fungus is obtained by crossing two parental fungal species, each of which having advantageous properties desirably to be combined in one fungal species. For instance, a fungal species having a relatively high growth rate may be crossed with a fungal species with the appropriate degradation capacity, for instance the capacity to degrade lignocellulosic biomass, such as wood.

The incubation of the substrate with the fungus is done under conditions conducive to growth and metabolism of the fungus. The incubation is best performed in dark, aerobic conditions, at a relative humidity of about 65-100%, at a temperature range from about 20 °C to about 35 °C, and preferably at a temperature range from about 21 °C to about 30 °C, and a CO₂ level of 5000 to 20,000 ppm. When using a fermentation unit as described herein, usually no specific requirements with respect to forced aeration, forced agitation and/or active cooling are necessary.

The cultivation time should be such to achieve at least partial degradation of the substrate. Typically, the incubation time may be from 4 to 12 weeks. A suitable end point for the fermentation process may be determined by various factors, among which desired specifications of the end product. If the biomass substrate contains contaminations, the residual contamination level of the fermentation product may for instance be a requirement determining the fermentation period.

When fungal fruiting bodies (mushrooms) are formed and protrude through the perforated walls of the unit, they conveniently may be harvested by cutting the protruding fruiting bodies from the substrate.

The fermentation product typically contains a solid fraction comprising fungal biomass and an insoluble residue of the biodegraded substrate, and a liquid fraction comprising soluble metabolites obtained from fungal metabolism, such as simple sugars (mono-, di- and oligosaccharides), amino acids, peptides, proteins, enzymes, vitamins, bioactive compounds.

Due to the biodegradation/bioremediation of the biomass-containing substrate as described herein, the resulting fermentation product advantageously has a more homogeneous composition and structure than the starting substrate. Various complex organic molecules present in the starting substrate are converted by fungal metabolism to far more simple degradation products. Complex fibrous material, for instance plant cell wall material like lignocellulosic or cellulosic material, is converted to fibrous material with improved structural and compositional features. In addition, pollutants, if present, are degraded. Finally, the composition and structure of the fermentation product can easily be standardized as desired, contrary to the starting substrate. Thus, due to its more homogeneous composition and structure, further processing of the fermentation product according to the wishes of the further user is much easier than when using the starting biomass.

The fermentation process may advantageously be adapted and fine tuned to obtain a fermentation product with desired specifications.

A particular method for solid state fermentation of a fungus on a substrate, the substrate comprising biomass, comprises:
a) bringing the substrate in the form of particles having a diameter of at the most 15 mm,
b) adjusting the moisture content of the substrate to obtain a dry matter content of 40-90 % (w/w),
c) inoculating the substrate with an inoculum of a fungus,
d) incubating the substrate with the fungus in the system for solid state fermentation as described in the previous aspect of this invention, for a time period to achieve at least partial degradation of the substrate,
e) further processing of the mixture of fungus and degraded substrate as desired.

Depending on the further use of the fermentation product, several options for further processing of the fermentation product are available.

In one embodiment, the fermentation product is separated in a liquid and a solid fraction, which each may be further processed as desired. Such separation may be done using conventional solid-liquid separation technology. If necessary, water or another suitable liquid or solvent may be added to the fermentation product to facilitate and/or improve the separation and/or further processing. Further processing of the liquid fraction for instance may comprise concentration, purification of components of interest, granulation and/or drying. Further processing of the solid fraction for instance may comprise shredding, grinding, drying, and/or compacting like granulation or pelletizing.

Further use of the fermentation product may comprise use as a source of a compound of interest, use as a component of a microbial cultivation medium, use for the generation of energy, e.g. as fuel for combustion ovens, use as animal feed, use for soil enhancement, and/or recycling by feeding it into an upstream step of the biodegradation process as described herein.

In particular, the solid fraction may be used as a bioremediation agent, for environment enhancing soil treatment 'in situ', as a biofilter in aqueous environments and/or watersheds, as a water retention device in, for example, low soil-humidity areas or in dykes to prevent flooding, as a raw material for paper and cardboard producing industries. The liquid fraction may be used in fermentation for multiple acid(s) production, as a feed for a microbial fuel cell, for derivation of energetic components for energy conversion, for heavy and precious metal recovery, for bionanotechnology applications.

## Claims

1. A system for solid state fermentation of a fungus, the system comprising a plurality of separate fermentation units placed next to each other, wherein the size of at least one dimension of each unit is accommodated to provide conditions for aerobic fermentation of the fungus without the need to install in the unit means for at least one of forced aeration, forced agitation and active cooling of the fungal culture.

2. The system of claim 1, wherein the size of the at least one dimension is at the most about 50 cm, preferably is about 5 to about 50 cm.

3. The system of claim 1, wherein each unit has a volume of about 10 to about 150,000 Liter.

4. The system of claim 1, wherein at least one wall of each unit is permeable to gas.

5. The system of claim 1, wherein each unit is rectangular and has a width of about 5 to about 50 cm, a height of about 5 cm to about 15 m and a length of about 5 cm to about 20 m.

6. The system of any one of the preceding claims, wherein each unit is configured for loading and/or unloading thereof, said loading and/or unloading being configured to occur when a unit occupies at least one specific position in the system.

7. The system of any one of the preceding claims, wherein the fermentation units are movable, preferably in an endless path.

8. A method for solid state fermentation of a fungus comprising use of the system of any one of the preceding claims.

9. The method of claim 8, wherein the fungus is cultivated on a substrate comprising biomass.

10. A method for the biodegradation of a substrate, wherein the substrate comprises biomass, the method comprising:
a) bringing the substrate in the form of particles having a diameter of at the most 15 mm,
b) adjusting the moisture content of the substrate to obtain a dry matter content of 40-90 % (w/w),
c) inoculating the substrate with an inoculum of a fungus,
d) incubating the substrate with the fungus in the system of any one of claims 1 to 7, for a time period to achieve at least partial degradation of the substrate,
e) further processing of the mass of fungus and degraded substrate as desired.

11. The method of claim 10, wherein the moisture content of the substrate is adjusted by adding a liquid comprising manure and/or liquid agricultural and/or industrial waste.

12. The method of claim 10 or 11, wherein the further processing comprises separating the mass of fungus and degraded substrate in a solid and a liquid fraction.

13. The method of claim 9 to 12, wherein the biomass comprises wood, crop residues, animal remains, municipal waste and/or agricultural waste.

14. The method of claim 13, wherein the biomass comprises biomass contaminated with inorganic or organic pollutants.
